# EUROPÄISCHE PATENTANMELDUNG

(11) **EP 1 266 605 A2**
(43) Veröffentlichungstag der Anmeldung: **18.12.2002**
(21) Anmeldenummer: 02012915.1
(22) Anmeldetag: 11.06.2002
(51) Int. Cl.: A61B 5/00

(54) **Integrierter Kabeladapter zum Anschluss an neurochirurgische Sensoren**

(30) Priorität: 13.06.2001 DE 20109864 U
(71) Anmelder: REHAU AG + Co, 95111 Rehau (DE)
(72) Erfinder: Stendel, Rüdiger, Dr., 14163 Berlin (DE); Gropp, Friedrich Dr., 12526 Berlin (DE); Göhler, Karlheinz, 08297 Zwönitz (DE)

(57) **Zusammenfassung**

Die Erfindung betrifft einen integrierten Kabeladapter zur Adaption an neurochirurgische Sensoren.

Der integrierte Kabeladapter ist dadurch gekennzeichnet, dass er eine unabhängige Stromversorgung mittels Akku oder Batterie sowie einer Anzeige durch Zahlendisplay aufweist und dass ein Alarmgeber und eine Nullpunktschaltung integriert sind. Der integrierte Kabeladapter zeichnet sich dadurch aus, dass ohne einen Monitoranschluss das Zahlendisplay in diesem ständig in Funktion ist und dass bei einem Monitoranschluss das Zahlendisplay nicht in Funktion ist.

## Beschreibung

Die Erfindung betrifft einen integrierten Kabeladapter zur Adaption an neurochirurgische Sensoren.

Über an Monitorsysteme angeschlossene in-vivo-Sensoren sind neurochirurgisch wichtige Parameter, wie intracranieller Druck, Sauerstoffsättigung, Temperatur o. ä. zugänglich.

Je nach Aufenthalt des Patienten, entweder im OP, bei der ambulanten Notfallversorgung, auf dem Transport oder auf der Station finden verschiedene Monitorsysteme Verwendung: Patienten-Monitore, Zwischen- oder Transportmonitore, Mess- und Eichmonitore usw., welche nur in Verbindung mit spezifischen Sensoren benutzt werden können.

Will man einen Sensor an verschiedenen Monitoren betreiben, lässt sich dies durch den Einsatz sogenannte Nullpunktsimulatoren erreichen, die es erlauben die Messwerte der Sensoren zuverlässig zu sichern.

Es macht nur Sinn, einen neurochirurgischen Sensor zu implantieren, wenn er zuvor über einen Nullpunktsimulator an einen vorhandenen Monitor adaptiert ist.
Oft sind allerdings, beispielsweise in der Notaufnahme oder im Rettungswagen, keine Monitore vorhanden, so dass der gewünschte Parameterwert, beispielsweise der intracranielle Druck, nicht verfügbar ist. Das kann für den Patienten, vor allem beim Transport, gefährlich werden, da sein Zustand nicht in geeigneter Weise überwacht und gegebenenfalls Notmaßnahmen ergriffen werden können.
Weiterhin muss ein einmal korrekt implantierter Sensor immer wieder vom Überwachungsmonitor abgekoppelt werden, z. B. wenn der Patient von der Station zum OP oder zur Untersuchung transportiert wird. Hier kann man sich im Einzelfall mit einem Transportmonitor behelfen, was jedoch einen zusätzlichen Geräteaufwand inklusive Bedienung und Platzbedarf bedeutet.

Außerdem: Wenn dann der Patient wieder auf Station ist und an den Patientenmonitor wieder angeschlossen ist, wird der intracranielle Druck an beiden Monitoren angezeigt, was zum einen unnötig ist, zum andern in der Praxis die Gefahr der Fehlbedienung mit sich bringt.
In manchen Fällen, beispielsweise im Computertomographen oder ähnlichen Geräten, ist der Anschluss eines Monitors unmöglich und damit der gewünschte Parameterwert nicht verfügbar.

Aufgabe der Erfindung ist es daher, diese Nachteile zu überwinden und für einen Sensor eine integrierte elektronische Einheit bereitzustellen, die eine monitor- und netzunabhängige Anzeige von Messparametern gewährleistet.

Erfindungsgemäß wird dieses Problem gelöst durch einen integrierten Kabeladapter mit einer unabhängigen Stromversorgung mittels Akku oder Batterie und mit einer Zahlendisplay- Anzeige.

Der integrierte Kabeladapter verfügt über eine unabhängige Stromversorgung durch Akku oder Batterie, die Anzeige der Batteriekapazität erfolgt über Leuchtdioden. Zusätzlich zu seiner Funktion als Nullpunktsimulator hat der Integrierte Kabeladapter eine Zahlendisplayanzeige, über die - wenn kein Monitor angeschlossen ist - der vom implantierten Sensor gelieferte, zu überwachende Parameter digital abzulesen ist. Dieser Wert bleibt durch Nullpunktsabgleich auch verfügbar, wenn das System an einem Monitor angeschlossen wird bzw. später von einem Monitor wieder abgekoppelt wird
Der integrierte Kabeladapter besitzt Schnittstellen für Datenübertragung per Funk und/oder Infrarot zur Übertragung des Alarmwertes an PCs oder Datenspeicher und/oder zur Auslösung eines Notrufes. Dazu lässt sich der zu überwachenden Parameter durch Bereichsgrenzen oder durch einen Einzelwert definieren.
Die Schaltungen sind so ausgeführt, dass jeweils nur eine Anzeige, entweder das Display des Integrierten Kabeladapters oder der Monitor, aktiv sein kann.

Der wichtigste Vorteil, den der erfindungsgemäße integrierte Kabeladapter bietet, ist die Möglichkeit, einen Sensor zur Messung, beispielsweise des intracraniellen Druckes, praktisch überall implantieren zu können: Der integrierte Kabeladapter hat keine äußere Stromversorgung nötig und so lässt sich sowohl der Nullpunktabgleich des zu implantierenden Sensors durchführen, als auch nach der Implantation der Zahlenwert des intracraniellen Druckes direkt ablesen.
Dies ist gerade beim Transport des Patienten, bei der ambulanten Notfallversorgung oder bei Untersuchungen mittels Computertomographie oder Magneto-Resonanztomographie wichtig. Die Vorgänge am und mit dem Patienten sind also durch Einsatz des integrierten Kabeladapters ohne zusätzlichen Aufwand diagnostisch gesichert, auch wenn kein Patientenmonitor zur Verfügung steht.

Befindet sich der Patient nach der Implantation anschließend auf der Station, lässt sich der integrierte Kabeladapter an einen herkömmlichen Patientenmonitor anschließen, wobei der Nullpunktabgleich über die jeweiligen Nulltasten erfolgt und anschließend sofort der intracranielle Druck am Patientenmonitor zur Verfügung steht, während die Displayanzeige am Integrierten Kabeladapter erlöscht.

Sollte einmal eine Entkopplung vom Patientenmonitor erforderlich werden, werden automatisch alle Funktionen des integrierten Kabeladapters umgehend wieder aktiv, so dass der intracranielle Druck wieder sicher angezeigt und so die Situation des Patienten dargestellt wird.

Vorteilhaft ist auch, dass alle auf der Station, in der Ambulanz oder im OP schon vorhandenen Monitore weiter voll genutzt werden können, lediglich spezifische Steckeradapter erforderlich sind, was erhebliche Kosten spart. Auch sind - in Verbindung mit dem Integrierten Kabeladapter - keine besonderen Schulungen oder Lernphasen des Krankenhauspersonals notwendig.

Im Folgenden wird der erfindungsgemäße integrierte Kabeladapter näher beschrieben. Dabei zeigen:
- Figur 1 -: Vorderansicht des integralen Kabeladapters
- Figur 2 -: Integration des integralen Kabeladapters in eine Sensor-Kette.

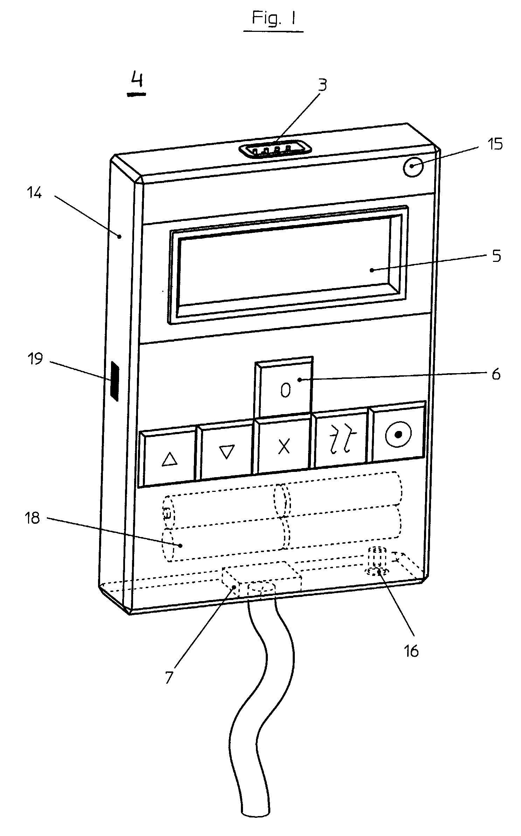

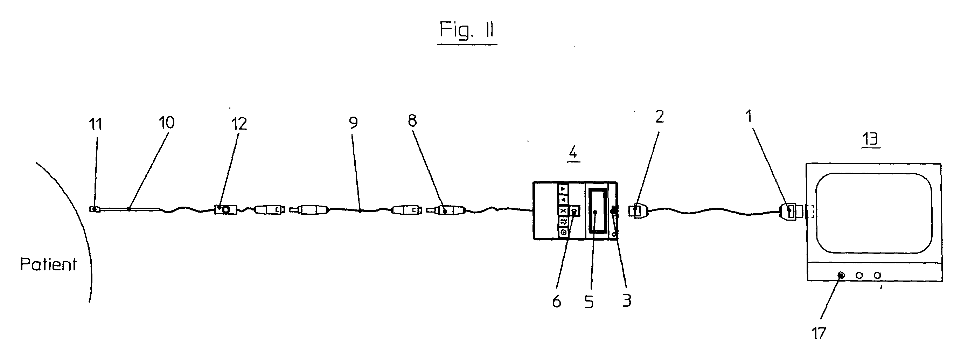

In Figur 1 ist der integrierte Kabeladapter 4 in seiner Vorderansicht dargestellt. In diesem Ausführungsbeispiel verfügt der integrierte Kabeladapter über ein rechteckiges Gehäuse 14 mit rechteckigem Querschnitt, an seinem freien oberen Ende über eine Steckerbuchse 3, die zum Anschluss an einen Patientenmonitor dient, über ein der Steckerbuchse 3 gegenüberliegendes elektronisches Element 7, über das die neurochirurgischen Parameter in den Integrierten Kabeladapter 4 eingebbar sind.
Der integrierte Kabeladapter 4 weist in seinem Inneren Batterien bzw Akkus 18 auf, die eine unabhängige Stromversorgung über einen längeren Benutzungszeitraum sicherstellen, sowie eine Steckerbuchse 16 für ein Ladegerät. Die Überwachung dieser Stromversorgung geschieht über eine separat eingebrachte Ladekontrollleuchte 15 für den Batterie- bzw Akku-Ladestand. Weiters ist im Sichtbereich des integrierten Kabeladapters ein Zahlendisplay 5 angebracht, welches die Patientendaten anzeigt. Zur Kalibrierung des integrierten Kabeladapters an das Sensorsystem über das elektronische Element 7 weist der Kabeladapter eine Nulltaste 6 auf. Zur Übertragung von Patientendaten an Speichereinheiten oder externe Alarmgeber verfügt der Kabeladapter über eine Infrarotschnittstelle 19.

Figur 2 zeigt die Sensor-Kette, bestehend aus dem Sensor 11, dem Kathederschlauch 10, der Stellschraube 12, dem Verlängerungskabel 9 und dem integralen Kabeladapter 4 mit Stecker 8.
Vor der Implantation wird der Sensor befeuchtet und der Nullabgleich durch Drehen der Stellschraube 12 herbeigeführt.
Dann wird der Sensor - einschließlich Kathederschlauch 10 und Verlängerungskabel 9 - durch Ziehen des Steckers 8 von 4 getrennt und anschließend implantiert.
Nach der Implantation wird die Sensorkette wieder komplettiert: Der intracranielle Druck wird am Zahlendisplay 5 angezeigt. Die Sensorkette kann nun gegebenenfalls neu unterbrochen und wieder verbunden werden, ohne Verlust der Messdaten bzw. der Messpräzision.

Wird der Adapterstecker 2 mit der Steckerbuchse 3 verbunden, erlischt die Messfunktion des integralen Kabeladapters. Es ertönt ein Warnton, bis der Monitorstecker 1 mit dem Patientenmonitor 13 verbunden ist. Der Nullpunktabgleich zwischen dem Patientenmonitor und der Sensorkette erfolgt nun, indem die Nulltasten 6 und 17 gleichzeitig gedrückt werden.
Alle Funktionen sind nun, wie gewohnt, am Patientenmonitor zu sehen.

Der erfindungsgemäße integrierte Kabeladapter lässt sich an alle üblichen, neurochirurgische Sensoren anschließen.

## Patentansprüche

1. Integrierter Kabeladapter für den Anschluss an neurochirurgische Sensoren **dadurch gekennzeichnet, dass**
der integrierte Kabeladapter (4) eine unabhängige Stromversorgung mittels Akku oder Batterie (18) sowie eine Anzeige durch Zahlendisplay (5) aufweist, weiterhin ein Alarmgeber und eine Nullpunktsschaltung integriert sind.

2. Integrierter Kabeladapter nach Anspruch 1, **dadurch gekennzeichnet, dass** - in Verbindung mit einem implantierten Sensor - ohne Monitoranschluss das Zahlendisplay in Funktion ist und dass mit Monitoranschluss das Zahlendisplay am Integrierten Kabeladapter nicht in Funktion ist

3. Integrierter Kabeladapter nach den Ansprüchen 1 und 2 **dadurch gekennzeichnet, dass** die Alarmfunktion im Falle von Fehlschaltungen, einer unvollständigen Messkette und/oder bei Fehlbedienung aktiviert wird.

4. Integrierter Kabeladapter nach den Ansprüchen 1 und 3, **dadurch gekennzeichnet, dass** digitale Eingabemöglichkeit der Alarmgrenzen per Schalterstellung als Bereichsgrenzen oder durch Einstellung als Einzelwert integriert sind.

5. Integrierter Kabeladapter nach einem der vorhergehenden Ansprüche, **dadurch gekennzeichnet, dass** für die Datenübertragung kabellose Schnittstellen vorhanden sind.

6. Integrierter Kabeladapter nach den Ansprüchen 1 bis 6, **dadurch gekennzeichnet, dass** in Verbindung mit einem spezifischen Kabelsatz der Anschluss aller gängigen Patienten-Monitore vorgesehen ist.
